**Europäisches Patentamt**

**European Patent Office**

**Office européen des brevets**

(11) Publication number: **0 121 569**
**B1**

## (12) EUROPEAN PATENT SPECIFICATION

(45) Date of publication of patent specification: **22.08.90**

(21) Application number: **83902695.2**

(22) Date of filing: **24.08.83**

(86) International application number:
**PCT/JP83/00276**

(87) International publication number:
**WO 84/01583 26.04.84 Gazette 84/11**

(51) Int. Cl.⁵: **C 12 N 15/00, C 07 H 21/04,
C 12 P 19/34, C 12 N 1/20 //
(C12N1/20, C12R1:19)**

(54) NOVEL VECTOR.

(30) Priority: **08.10.82 JP 177192/82
28.01.83 JP 122/82**

(43) Date of publication of application:
**17.10.84 Bulletin 84/42**

(45) Publication of the grant of the patent:
**22.08.90 Bulletin 90/34**

(84) Designated Contracting States:
**AT BE CH DE FR GB LI LU NL SE**

(56) References cited:
**EP-A-0 041 767    EP-A-0 118 617**
**EP-A-0 048 970    FR-A-2 511 033**
**EP-A-0 052 002    JP-A-5 838 300**
**EP-A-0 083 069    JP-A-5 892 695**
**EP-A-0 091 539**

**DNA, vol. 2, no. 4, 1983, pages 265-273, Mary
Ann Liebert, Inc. Publishers; T. NISHI et al.:
"Construction and application of a novel
plasmid "ATG vector" for direct expression of
foreign genes in Escherichia coli"**

(73) Proprietor: **KYOWA HAKKO KOGYO CO., LTD.
6-1, Ohte-Machi 1-chome
Chiyoda-ku Tokyo-to (JP)**

(73) Proprietor: **JURIDICAL FOUNDATION
JAPANESE FOUNDATION FOR CANCER
RESEARCH
37-1, Kamiikebukuro 1-chome Toshima-ku
Tokyo 170 (JP)**

(72) Inventor: **TANIGUCHI, Tadatsugu
Yuparesu Tagara 303 27-12 Tagara 4-chome
Nerima-ku Tokyo 176 (JP)**
Inventor: **SUGANO, Haruo
8-13, Minamiogikubo 4-chome
Suginami-ku Tokyo 167 (JP)**
Inventor: **NISHI, Tatsunari
6-6, Asahi-machi 3-chome
Machida-shi Tokyo 194 (JP)**
Inventor: **SATO, Moriyuki
12-2, Asahi-machi 1-chome
Machida-shi Tokyo 194 (JP)**
Inventor: **ITO, Seiga
218-14, Hachimannishi Aihara
Sagamihara-shi Kanagawa 229 (JP)**

(74) Representative: **Vossius & Partner
Siebertstrasse 4 P.O. Box 86 07 67
D-8000 München 86 (DE)**

# EP 0 121 569 B1

**Description**

The present invention relates to a novel plasmid expression vector, a process for producing the same and its use.

More specifically, the present invention relates to an expression vector having a promoter, a ribosome binding site, an initiation codon for translation and a recognition sequence of a restriction enzyme to be left as 3'-protruding ends after cleavage with the restriction enzyme, which is characterized in that the recognition sequence is situated immediately downstream of the initiation codon. Furthermore, the present invention relates to a process for producing such an expression vector and to its use.

In recent years, research and technology on recombinant DNA have been developed and the industrial applicaton thereof has become possible. It is therefore important to develop a process for incorporating a foreign gene into a plasmid vector and for synthesizing effectively a polypeptide encoded by the foreign gene in a host microorganism. Various methods have been developed to effectively express foreign genes in host microorganisms such as *Escherichia coli*. In order to elevate the transcription rate, the lac promoter, trp promoter, etc. were used as a promoter which is the genetic information necessary for the initiation of transcription. Further, in order to elevate the translation rate, a recombinant plasmid was constructed wherein the distance between the ribosome binding site and the initiation codon for translation (mainly ATG) was adjusted.

The latter is disadvantageous because in order to adjust the distance between the ribosome binding site and the initiation codon the use of a special "synthetic DNA" as a linker to incorporate a foreign gene after attaching the initiation codon ATG downstream from the promoter, the use of DNA modification enzymes, such as DNA polymerase I, S1 nuclease and BAL-31 nuclease, or the use of a "synthetic DNA" having a special base sequence is required.

EP—A 41 767 describes the expression plasmid pPLc24 which contains the $P_L$ promoter, a ribosome binding site, an initiation codon for translation, and the codons for the first 98 amino acid residues of the bacteriophage MS2 replicase gene. A restriction site is located downstream of said 98 codons. Thus, all expression products which can be obtained by using plasmid pPLc24 are fusion proteins.

In order to improve the known plasmid vectors, the present inventors have developed an expression vector in which the recognition sequence of a restriction enzyme creating 3'-protruding ends is situated immediately downstream of an initiation codon.

The present invention is described in detail below and in the attached claims.

The expression vector of the present invention contains a promoter which is the genetic information necessary for the initiation of transcription, an initiation codon for translation (ATG or GTG) residing at an appropriate position downstream from the ribosome binding site which is the genetic information necessary for the initiation of translation and which is named the Shine Dalgarno sequence, abbreviated as "SD sequence", in the case of *Escherichia coli*, and a recognition sequence of a restriction enzyme, such as SphI, KpnI or SacI, to be left as 3'-protruding ends after cleavage with the restriction enzyme following the initiation codon.

As the promoter of the expression vector of the present invention, any promoters having promoter activity are applicable. Preferably, the promoter of the lactose operon, that of the tryptophan operon and that of the alkaline phosphatase operon are used. ATG or GTG are used as the initiation codon for translation. Translation is possible when the distance between the SD sequence and the initiation codon is 2—20 base pairs (bp). Examples of the recognition sequence of a restriction enzyme to be left as 3'-protruding ends following the initiation codon are ATGCATGC wherein the initiation codon ATG and the recognition sequence of SphI overlap, ATGGTACC wherein ATG and the recognition sequence of KpnI overlap, and ATGAGCTC wherein ATG and the recognition sequence of SacI overlap.

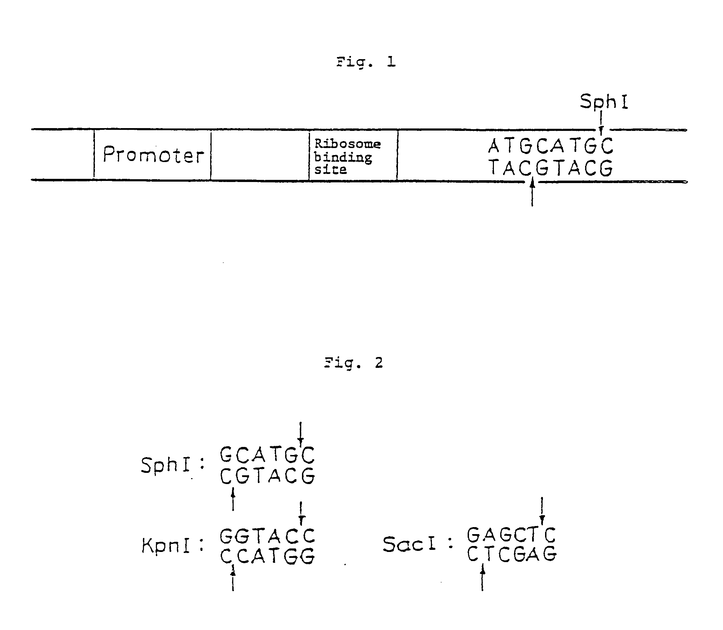

The vector wherein the initiation codon ATG and the SphI cleavage site (GCATGC) overlap is selected as an example of a vector having the structure mentioned above and the scheme of the structure around the promoter and the SD sequence is illustrated in Fig. 1. Further, cleavage patterns of the restriction enzymes mentioned above, the recognition site of which overlap with the initiation codon ATG, are illustrated in Fig. 2.

The construction of the plasmid vector of the present invention is described in detail using the tryptophane promoter of *Escherichia coli* as an example.

In order to facilitate the understanding of the present invention, the following description is made mainly with reference to *Escherichia coli* which is a prokaryote clarified in detail as to the mechanism of transcription and translation.

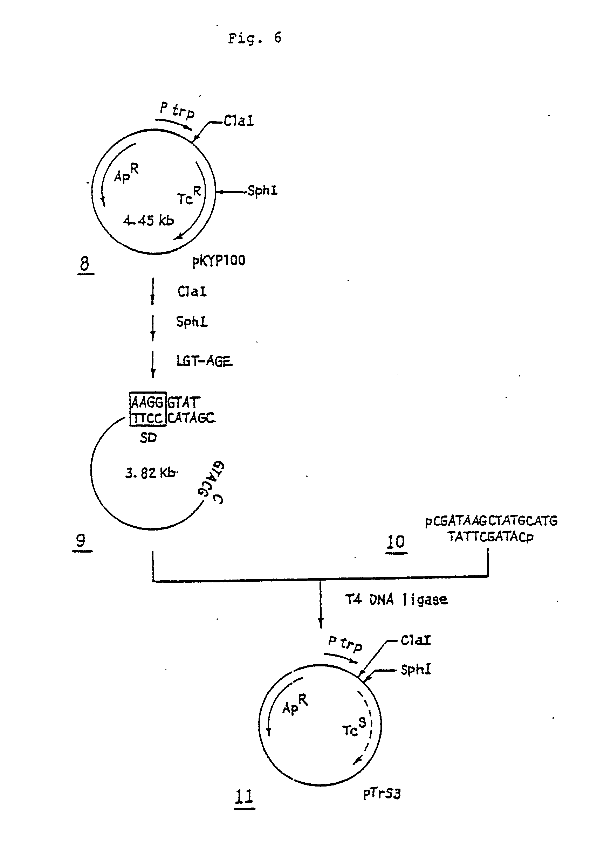

As a source of a DNA containing the trp promoter, pKYP100 is used which is a derivative of pKYP10 bearing a trp portable promoter (Japanese Patent Application No. 213193/81, U.S. Patent Application Serial No. 452,290, European Patent Application No. 82111895.7). The methods of producing pKYP10 and pKYP100 are described in Reference Examples 1 and 2 below. pKYP100 *8* (*8* refers to the number in Fig. 6, the same shall apply hereinafter) is cleaved at the ClaI site existing just downstream from the SD sequence of the trpL gene (see Fig. 6). pKYP100 is then cleaved again at the SphI site existing in the tetracycline resistance gene and the larger plasmid DNA fragment *9* is purified by agarose gel electrophoresis.

Separately, two oligonucleotides are synthesized which have complementary nucleotide sequences ligatable with each other to make a DNA fragment *10* which has a structure ligatable with the DNA fragment

2

9 and an initiation codon for translation (ATG or GTG) at an appropriate distance downstream from the SD sequence followed by a recognition sequence of a restriction enzyme to be left as 3'-protruding end. The thus obtained DNA fragment is ligated with the purified plasmid DNA fragment 9 to obtain the desired expression vector 11.

Reaction conditions required for preparing the recombinant plasmid are as follows.

Digestion of the DNA with restriction enzymes is usually carried out by reacting 0.1 to 100 µg of DNA with 0.1—300 units, preferably 1—3 units of restriction enzyme per 1 µg of DNA in a mixture of 2—200 mM, preferably 10—40 mM Tris-HCl (pH 6.0—9.5, preferably pH 7.0—8.0), 1—150 mM NaCl and 2—20 mM, preferably 5—10 mM $MgCl_2$ at 18 —42°C, preferably 32—38°C for 15 minutes to 24 hours. The reaction is usually stopped by heating at 55—75°C, preferably 63—70°C for 5—30 minutes, or alternatively by inactivating the restriction enzyme with a reagent such as phenol or diethylpyrocarbonate. Oligonucleotides are synthesized by the diethyl phosphate method [H. G. Khorana et al.: J. Mol. Biol., 72, 209 (1972)], the phosphotriester method [R. Crea et al.: Proc. Natl. Acad. Sci., U.S.A., 75, 5765 (1978)] or the phosphite method [M.D. Matteucci et al.: J. Am. Chem. Soc., 103, 3185 (1981)]. Phosphorylation of the synthesized oligonucleotides is carried out with 0.1—100 units of T4 polynucleotide kinase in a mixture of 2—200 mM, preferably 10—70 mM Tris-HCl (pH 6.0—9.5, preferably pH 7.0—8.0), 3—20 mM, preferably 4—10 mM $MgCl_2$ and 1—10 mM dithiothreitol at 20—40°C, preferably 35—38°C for 5 minutes to 2 hours. The ligation of the DNA fragments is carried out with 0.1—10 units of T4 DNA ligase in a mixture of 2—200 mM, preferably 10—70 mM Tris-HCl (pH 6.0—9.5, preferably 7.0—8.0), 2—20 mM, preferably 5—10 mM $MgCl_2$, 0.1—10 mM, preferably 0.5—2 mM ATP and 1—50 mM, preferably 5—10 mM dithiothreitol at 1—37°C, preferably 3—20°C for 15 minutes to 72 hours, preferably 2—20 hours.

The plasmid expression vector of the present invention can be prepared in the manner described above. Alternatively, instead of the cleavage of pKYP100 with ClaI, it is possible to cleave pKYP100 at the HindIII site just downstream from the ClaI site and then at the SphI site to insert the synthetic oligonucleotide. When the recognition sequence of the restriction enzyme overlapping with the initiation codon for translation is absent on pKYP100, for example,

$$\downarrow \qquad \qquad \downarrow$$
KpnI (GGTACC) and SacI (GAGCTC),

two species of oligonucleotides including such recognition sequence are synthesized and inserted into pKYP100 between the ClaI site or HindIII site and BamHI site or SalI site.

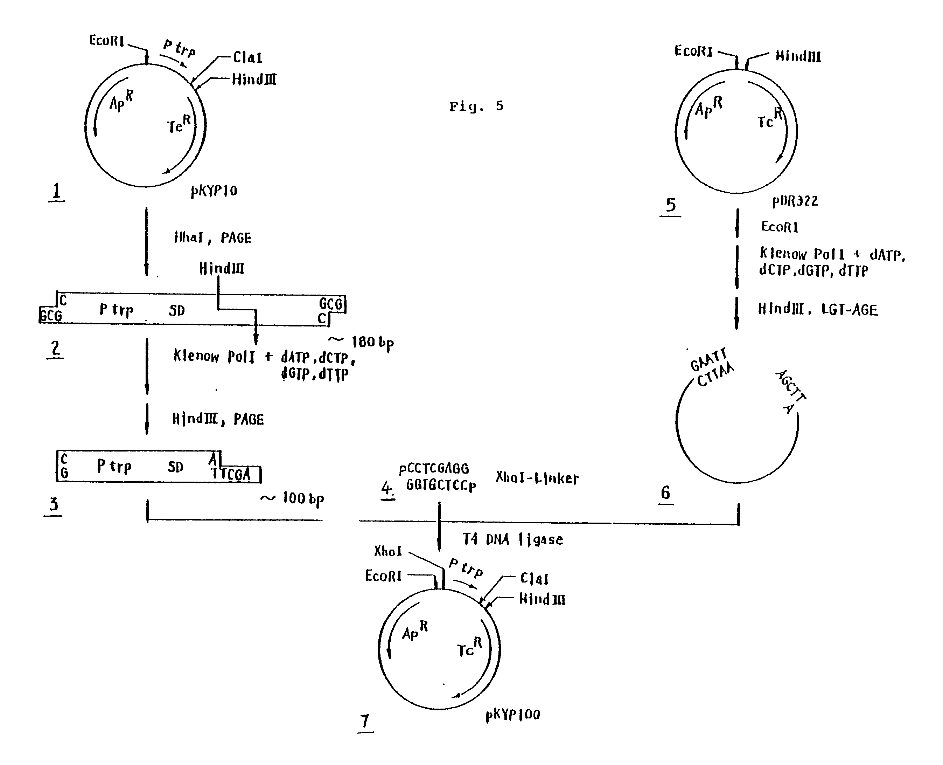

pKYP100 is a derivative of pKYP10. pKYP10 is a plasmid containing a trp promoter. It is described in the Japanese Patent Application No. 213193/81. The construction of pKYP100 from pKYP10 is carried out in the following manner. As illustrated in Fig. 5, plasmid pKYP10 1 is digested with HhaI and a DNA fragment 2 of about 180 bp containing a trp promoter is purified by polyacrylamide gel electrophoresis. The DNA fragment 2 is treated with Escherichia coli DNA polymerase I · Klenow fragment to chew back 3'-protruding ends formed by a HhaI digestion in order to convert them into flush ends. After digestion with HindIII, a DNA fragment 3 of about 100 bp containing a trp promoter is purified by polyacylamide gel electrophoresis.

Separately, plasmid pBR322 5 is digested with EcoRI and the resulting sticky ends are converted into flush ends by a repairing synthesis reaction with Escherichia coli DNA polymerase I. After digestion with HindIII, the larger plasmid DNA fragment 6 is purified by low-gelling-temperature agarose gel electrophoresis. The purified DNA fragments 3 and 6 are ligated with the 5'-phosphorylated XhoI linker (pCCTCGAGG) 4 using T4 DNA ligase to obtain plasmid pKYP100 7.

In the production of the above-mentioned vector of the present invention, a cleavage site existing just downstream from the SD sequence is used. However, even if such a cleavage site is absent, the desired expression vector can also be produced. For example, when a cleavage site existing upstream from the promoter is used, an oligonucleotide containing all of the promoter, ribosome binding site and initiation codon for translation (ATG or GTG) is synthesized and cloned in a plasmid vector such as pBR322. The tryptophan promoter of Escherichia coli used as a promoter for the initiation of transcription can be replaced by other Escherichia coli promoters. In the case where organism cells other than Escherichia coli are used for the expression of foreign genes, a promoter suitable for the cell is used. When a promoter of organisms other than Escherichia coli is used, it is necessary to replace the nucleotide sequence corresponding to the ribosome binding site of Escherichia coli by a nucleotide sequence containing the genetic information required for the initiation of translation.

The expression vector obtained as above can be cleaved with restriction enzymes such as SphI, KpnI and SacI and can be used to insert a foreign gene just after the initiation codon by suitable means, for example,

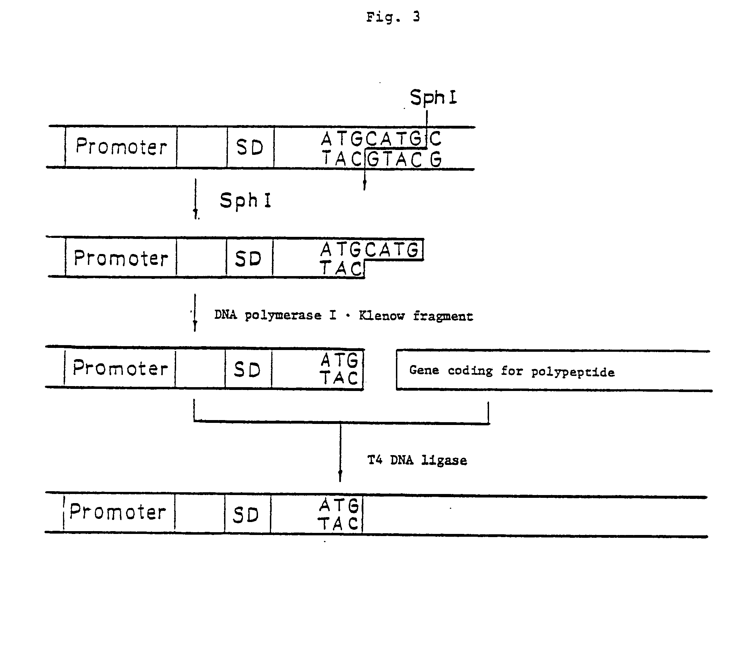

(1) by converting the 3'-protruding ends to flush ends using the 3' → 5' exonuclease activity of the DNA polymerase I · Klenow fragment followed by the insertion of the foreign gene (see Fig. 3) or

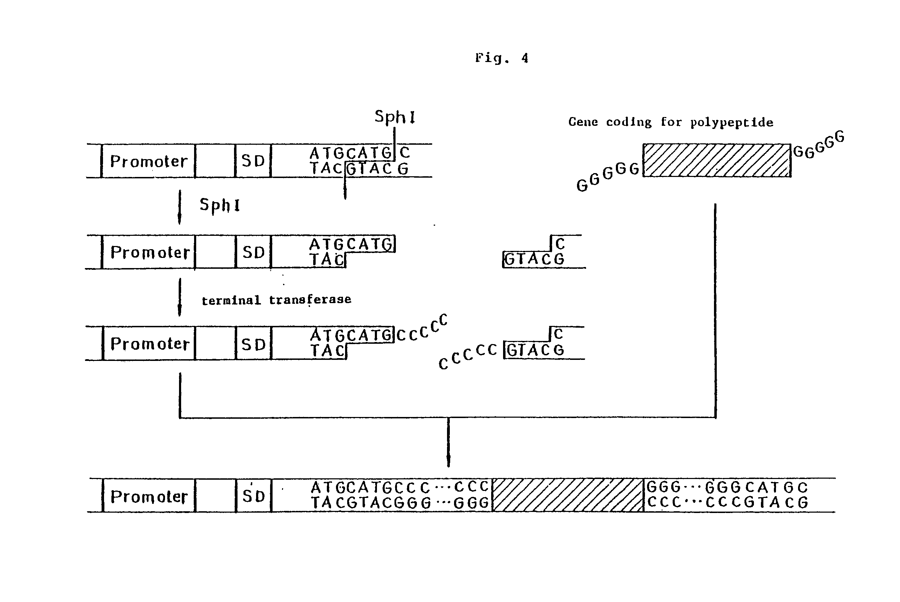

(2) by attaching a homopolymer block (polydA, polydT, polydG or polydC) to the 3'-protruding end using terminal deoxynucleotidyl transferase followed by the insertion of the foreign gene with a homopolymer block complementary to that mentioned above (see Fig. 4). The foreign gene can be expressed effectively by culturing a host microorganism containing the thus obtained recombinant.

3

The foregoing description refers to *Escherichia coli* for which molecular biological studies are most advanced. However, the use of the present invention is not restricted to *Escherichia coli* but also applicable to prokaryotes other than *Escherichia coli* and eukaryotes such as yeast and higher animals. That is, a plasmid expression vector applicable to other prokaryotes such as *Bacillus subtilis* can be constructed by maintaining the structure downstream from the initiation codon, replacing the promoter and the ribosome binding site of *Escherichia coli* with those of the respective prokaryote and attaching genetic information required for the replication and maintenance of the plasmid vector in the respective prokaryote. Further, although the mechanisms of transcription and translation in eukaryotes are not clarified in detail, the plasmid expression vector applicable to the eukaryotes can be constructed by replacing the promoter of *Escherichia coli* with genetic information for the initiation of transcription and the ribosome binding site of *Escherichia coli* with genetic information for the initiation of translation and attaching genetic information required for the replication and maintenance of the plasmid vector in the eukaryotes.

Fig. 1 is a scheme of one of the vectors of the present invention wherein the initiation codon for translation ATG and the SphI recognition site (GCATGC) overlap.

Fig. 2 shows the restriction sites suitable for the vector of the present invention.

Fig. 3 shows the process for inserting a foreign gene into the vector of the present invention using DNA polymerase I·Klenow fragment.

Fig. 4 shows the process for inserting a foreign gene into the vector of the present invention using terminal transferase.

Fig. 5 shows the process for constructing plasmid pKYP100. In the drawing, *1* refers to plasmid pKYP10, *2* refers to the DNA fragment of about 180 bp containing the trp promoter, *3* refers to the DNA fragment of about 100 bp containing the trp promoter, *4* refers to XhoI linker, *5* refers to plasmid pBR322, *6* refers to a DNA fragment of pBR322 and *7* refers to plasmid pKYP100.

Fig. 6 shows the process for constructing plasmid pTrS3. In the drawing, *8* refers to plasmid pKYP100, *9* refers to the fragment of about 3.82 Kb of pKYP100, *10* refers to double stranded DNA formed by the annealing of two synthesized oligonucleotides and *11* refers to plasmid pTrS3.

Fig. 7 shows the process for constructing pKYP5.

Fig. 8 shows the process for constructing pKYP10.

Fig. 9 shows the process for constructing pFJ123.

Fig. 10 shows the process for constructing pFM9.

Fig. 11 (A) shows the process for constructing pKYP11 and (B) the structure of pKYP12.

Fig. 12 shows the process for constructing pLE3.

The methods of constructing and utilizing the vector of the present invention are described in detail in the following examples.

## Example 1

Construction of plasmid vector pTrS3 bearing the initiation codon for translation ATG and an SphI cleavage site downstream from the trp promoter and the ribosome binding site

pTrS3 was obtained from pKYP100 constructed as in Reference Example 2 in the following manner. 5 µg of pKYP100 *8* was allowed to react with 5 units of ClaI (product of Boehringer Mannheim GmbH) in 20 µl of a reaction solution containing 10 mM Tris-HCl (pH 7.5), 7 mM $MgCl_2$ and 6 mM 2-mercaptoethanol at 37°C for 2 hours. The reaction was stopped by heating at 65°C for 5 minutes. 2 µl of 100 mM Tris-HCl (pH 7.5), 70 mM $MgCl_2$, 1.0 M NaCl, 60 mM 2-mercaptoethanol, 16 µl of distilled waer and 7 units of SphI (product of Boehringer Mannheim GmbH) were added and the mixture was allowed to react at 37°C for 2 hours. The reaction was stopped by heating at 65°C for 5 minutes and the larger plasmid DNA fragment *9* (about 3.82 Kb) was purified by low-gelling-temperature agarose gel electrophoresis (LGT—AGE) [Lars Wieslander: Analytical Biochemistry *98*, 305 (1979)].

Separately, two species of oligonucleotides 5'-CGATAAGCTATGCATG-3' and 5'-CATAGCTTAT-3' were synthetized by the phosphotriester method. The two synthesized oligonucleotides were 5'-phosphorylated and 20 µM each of the oligonucleotides were mixed with 10 mM Tris-HCl (pH 7.5), 100 mM NaCl and 1 mM EDTA. The mixture was incubated at 65°C for 10 minutes, at 37°C for 120 minutes and at room temperature for 120 minutes to anneal them. The two DNA chains were annealed as illustrated below.

pCGATAAGCTATGCATG

TATTCGATACp

Both ends of the resulting DNA fragment can be ligated with the DNA fragment having sticky ends formed by digestion with ClaI or SphI and the ligated DNA has a reconstituted ClaI cleavage site or SphI cleavage site. The annealed DNA *10* of the two oligonucleotides and the plasmid DNA fragment *9* purfied as above were mixed and ligated with T4DNA ligase. That is, 1 pM each of the two oligonucleotides, pCGATAAGCTATGCATG and pCATAGCTTAT were annealed and about 0.15 µg of the purified plasmid DNA fragment *9* was added. Then, 0.5 units of T4 DNA ligase (product of Takara Shuzo Co.) was added and the mixture was allowed to react in 20 µl of a reaction solution containing 20 mM Tris-HCl (pH 7.6), 10 mM $MgCl_2$, 10 mM dithiothreitol and 0.5 mM ATP at 4°C for 16 hours.

*Escherichia coli* HB101 was transformed with the resulting recombinant plasmid DNA. Plasmid DNA was isolated from the thus obtained transformant resistant to ampicillin and sensitive to tetracycline (Ap$^R$Tc$^S$) and purified. This plasmid DNA was digested with EcoRI, XhoI and PstI (products of Takara Shuzo Co.), and ClaI and SphI (products of Boehringer Mannheim GmbH) to determine the formation of the desired plasmid vector pTrS3 *11*. It was recognized by the method of Maxam and Gilbert [A. M. Maxam *et al.*: proc. Natl. Acad. Sci. *74*, 560 (1977)] that the nucleotide sequence of the DNA between the ClaI site and SphI site of pTrS3 was ATCGATAAGCTATGCATGC. *Escherichia coli* containing pTrS3 was deposited with the Fermentation Research Institute, Agency of Industrial Science and Technology (Ibaraki-ken, Japan) on September 29, 1982 as *Escherichia coli* lTrS-3 FERM P-6735. The deposit was transferred to an international deposit on July 26, 1983 and assigned accession number FERM BP-328.

Example 2

Expression of an interferon-β (referred to as IFN-β hereinafter) derivative wherein an additional methionine is attached to the N-terminus of IFN-β

A recombinant plasmid expressing an IFN-β derivative wherein an additional methionine was attached to IFN-β before the N-terminal methionine was obtained in the following manner using the expression vector pTrS-3 constructed in Example 1.

10 μg of pTrS-3 was reacted with 10 units of SphI (product of Boehringer Mannheim GmbH) in 40 μl of a reaction solution containing 10 mM Tris-HCl (pH 7.5), 50 mM NaCl, 7 mM MgCl$_2$ and 6 mM 2-mercaptoethanol at 37°C for 2 hours. After the reaction, the reaction mixture was subjected to phenol and chloroform extraction and ethanol precipitation. The precipitated DNA fragment was dissolved in 40 μl of a mixture containing 50 mM Tris-HCl (pH 7.6), 7 mM MgCl$_2$, 10 mM 2-mercaptoethanol, 0.25 mM dATP, 0.25 mM dCTP, 0.25 mM dGTP and 0.25 mM dTTP followed by addition of 4 units of *Escherichia coli* DNA polymerase I · Klenow fragment (product of Bethesda Research Laboratories Inc.). The mixture was allowed to react at 15°C for 2 hours. After the reaction, the mixture was subjected to phenol and chloroform extraction and ethanol precipitation. The precipitated DNA fragment was dissolved in 30 μl of a mixture containing 100 mM Tris-HCl (pH 7.5), 50 mM NaCl, 7 mM MgCl$_2$ and 6 mM 2-mercaptoethanol followed by addition of 16 units of EcoRI (product of Takara Shuzo Co.). The mixture was allowed to react at 37°C for 2 hours. After digestion with EcoRI, the smaller plasmid DNA fragment (about 130 bp) was purified by low-gelling-temperature agarose gel electrophoresis.

Then, 5 μg of a recombinant plasmid pLE-3 bearing the IFN-β gene was reacted with 10 units of ClaI (product of Boehringer Mannheim GmbH) in 30 μl of a reaction solution containing 10 mM Tris-HCl (pH 7.5), 7 mM MgCl$_2$ and 6 mM 2-mercaptoethanol at 37°C for 2 hours.

The structure around the ClaI site of pLE-3 is as follows.

```
                        ClaI

    A A A|A A G G|G T A T|C G A T G A G C T A C ....
    T T T|T T C C|C A T A G C|T A C T C G A T G
                              └──┘ └──┘ └──┘
        SD                    Met  Ser  Tyr
```

The reaction mixture was subjected to phenol and chloroform extraction and ethanol precipitation. The precipitated DNA fragment was dissolved in 30 μl of a mixture containing 50 mM Tris-HCl, 7 mM MgCl$_2$, 10 mM 2-mercaptoethanol, 0.25 mM dATP and 0.25 mM dTTP followed by addition of 12 units of *Escherichia coli* DNA polymerase I (product of new England Biolabs). The mixture was allowed to react at 37°C for 30 minutes.

By the reaction, 5'-protruding end formed by ClaI digestion was changed to flush end by 5' → 3' exonuclease activity of DNA polymerase I.

```
          ClaI

        A T|C G A T
        T A G C|T A

            ⇓

        A T    A T
        T A    T A
```

In the reaction, dATP and dTTP were added to prevent the digestion of the end of the DNA in the 3' → 5' direction by the 3' → 5' exonuclease activity of DNA polymerase I. The flush end mentioned above was obtained in a ratio of 2—20%.

The reaction solution was subjected to phenol and chloroform extraction and ethanol precipitation. The precipitated DNA fragment was dissolved in 30 μl of a mixture containing 100 mM Tris-HCl (pH 7.5), 50 mM NaCl, 7 mM MgCl$_2$ and 6 mM 2-mercaptoethanol and 16 units of EcoRI (product of Takara Shuzo Co.) was added. The mixture was allowed to react at 37°C for 2 hours.

After the digestion with EcoRI, the larger plasmid DNA fragment (about 5.1 Kb) was purified by low-gelling-temperature agarose gel electrophoresis. About 40 ng of the DNA fragment of about 130 bp containing the trp promoter, the SD sequence and the ATG codon and about 150 ng of the DNA fragment of about 5.1 Kb containing IFN-β gene thus obtained were reacted with 1 unit of T4 DNA ligase in 20 μl of a mixture of 20 mM Trs-HCl (pH 7.6), 10 mM MgCl$_2$, 10 mM dithiothreitol and 0.5 mM ATP at 4°C for 20 hours.

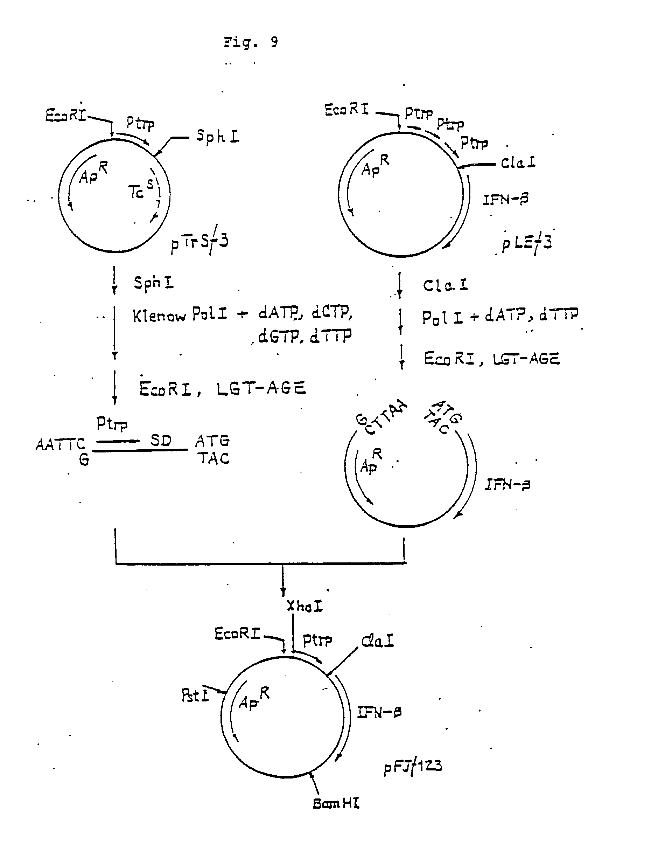

*Escherichia coli* HB101 [H. Boyer *et al.*: J. Molec. Biolo., *41*, 459 (1969)] was transformed with the thus obtained recombinant plasmid DNA in a conventional manner. The DNA of plasmid pFJ123 was isolated from an Ap$^R$Tc$^S$ transformant strain and purified in a conventional manner as illustrated in Fig. 9.

The structure of pFJ123 was determined by agarose gel electrophoresis after digestion with the restriction enzymes EcoRI, Xhol, Pstl and BamHl (products of Takara Shuzo Co.) and Clal (product of Boehringer Mannheim GmbH).

*Escherichia coli* HB101 containing recombinant plasmid pFJ123 was named IFJ-123. Interferon productivity of IFJ-123 was examined in the following manner.

The strain was cultured at 37°C for 18 hours in LG medium containing 10 g of tryptone, 5 g of yeast extract, 5 g of NaCl, 1 g of glucose and 1 l of water and adjusted to pH 7.2 with NaOH. 0.2 ml of the cultured medium was inoculated in 10 ml of MCG medium (pH 7.2) containing 0.6% Na$_2$HPO$_4$, 0.3%, KH$_2$PO$_4$, 0.5% NaCl, 0.1% NH$_4$Cl, 0.5% glucose, 0.5% casamino acid, 1 mM MgSO$_4$ and 4 μg/ml thiamine hydrochloride and cultured at 30°C for 4—8 hours. 10 μg/ml indole acrylic acid (referred to as IAA hereinafter) which is an inducer of the tryptophane gene was added and the culturing was continued for 5—12 hours.

The cultured medium was subjected to centrifugation at 8,000 rpm for 10 minutes and the harvested cells were washed with a buffer solution (pH 7.5) of 30 mM Tris-HCl containing 30 mM NaCl. The washed cells were suspended in 1 ml of the buffer solution mentioned above and 200 μg of lysozyme and 5 μl of 0.25 M EDTA (ethylenediamine tetraacetate) were added. The mixture was allowed to stand at 0°C for 30 minutes. Freezing and melting were repeated three times to disrupt the cells. The disrupted cells were subjected to centrifugation at 15,000 rpm for 30 minutes and the supernatant was recovered. The interferon in the supernatant was determined according to the method of Armstrong [J. A. Armstrong: Appl. Microbiol. *21*, 723—725 (1971)] using Vesicular Stomatitis Virus as a virus and Wish Cells derived from human amnion cells as an animal cell. The determination of interferon revealed that the strain produced 3 × 10$^9$ units/l interferon.

The base sequence around the SD sequence and the ATG in plasmid pFJ123 of the strain IFJ-123 as determined by the method of Maxam and Gilbert is as follows.

$$\underline{A\ A\ G\ G\ G}\ T\ A\ T\ C\ G\ A\ T\ A\ A\ G\ C\ T\ \underline{A\ T\ G}\ A\ T\ G$$

$$S\ D$$

*Escherichia coli* containing pFJ123 was deposited with the Fermentation Research Institute, Agency of Industrial Science and Technology on January 24, 1983 as *Escherichia coli* IFJ-123 FERM P-6882. The deposit was transferred to the international deposit on September 1, 1983 and assigned accession number FERM BP-346.

The above mentioned process for producing pFJ-123 is illustrated in Fig. 9.

### Example 3
#### Expression of an IFN-β derivative wherein N-terminal amino acids are deleted

A recombinant plasmid expressing an IFN-β derivative wherein several N-terminal amino acids are deleted was obtained in the following manner using the expression vector pTrS3 constructed in Example 1.

15 μg of pLE3 was digested with 20 units of Clal (product of Boehringer Mannheim GmbH) in 100 μl of a reaction solution containing 10 mM Tris-HCl (pH 7.5), 7 mM MgCl$_2$ and 6 mM 2-mercaptoethanol at 37°C for 2 hours. The reaction solution was subjected to phenol and chloroform extraction and ethanol precipitation. The precipitated DNA fragment was dissolved in 30 μl of 10 mM Tris-HCl (pH 7.5)—0.5 mM EDTA. 6 μl of the DNA solution containing about 3 μg of DNA, 13 μl of distilled water and 5 μl of buffer solution containing 60 mM CaCl$_2$, 60 mM MgCl$_2$, 3 M NaCl, 100 mM Tris-HCl (pH 8.1) and 5 mM EDTA were mixed and 0.5 units of exonuclease Bal31 (product of New England Biolabs) were added. The mixture was allowed to react at 30°C for 3 minutes to remove 1—30 bp from both Clal ends of the DNA. The reaction was stopped by phenol extraction and subsequently chloroform extraction and ethanol precipitation were carried out. The precipitated DNA fragment was dissolved in 20 μl of a mixture of 100 mM Tris-HCl (pH 7.5), 50 mM NaCl, 7 mM MgCl$_2$ and 6 mM 2-mercaptoethanol and 8 units of EcoRI (product of Takara Shuzo Co.) were added. The mixture was allowed to react at 37°C for 2 hours. After the digestion with EcoRI, the larger plasmid DNA fragment of about 5.1 Kb was purified by low-gelling-temperature agarose gel electrophoresis.

About 150 ng of the thus obtained DNA fragment of about 5.1 Kb containing the IFN-β gene and about 40 ng of the DNA fragment of about 130 bp prepared in Example 2 and containing the trp promoter, SD

6

sequence and the ATG codon were ligated with 1 unit of T4 DNA ligase (product of Takara Shuzo Co.) in 20 µl of a reaction solution containing 20 mM Tris-HCl (pH 7.6), 10 mM MgCl$_2$, 10 mM dithiothreitol and 0.5 mM ATP. The mixture was allowed to react at 4°C for 20 hours.

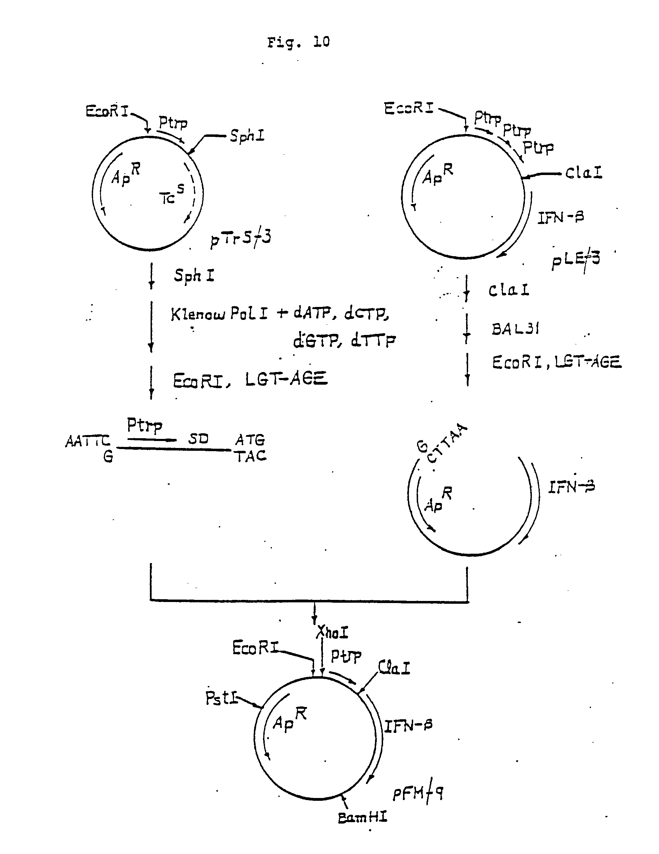

*Escherichia coli* HB101 was transformed with the thus obtained recombinant plasmid DNA in a conventional manner. The plasmid pFM9 was isolated from the obtained Ap$^R$Tc$^S$ transformant and purified in a conventional manner as illustrated in Fig. 10.

The structure of pFM9 was determined by agarose gel electrophoresis after the digestion with restriction enzymes EcoRI, XhoI, PstI, BamHI and ClaI.

*Escherichia coli* HB101 containing the recombinant plasmid pFM9 was named IFM-9. Interferon Productivity of IFM-9 was examined as in Example 2. The strain produced $8 \times 10^7$ units/l interferon. The strain was deposited with the Fermentation Research Institute, Agency of Industrial Science and Technology on January 24, 1983 as *Escherichia coli* IFM-9 FERM P-6883. The deposit was transferred to the international deposit on September 1, 1983 and assigned accession number FERM BP—347. The recombinant plasmid contained in this strain was named pFM9.

The above mentioned process for producing pFJ123 is illustrated in Fig. 10.

The base sequence around the SD sequence and the ATG in the plasmid pFM9 of *Escherichia coli* IFM-9 as determined by the method of Maxam and Gilbert is as follows.

A A G G G T A T C G A T A A G C T A T G G A T T C

S D

It was confirmed that pFM-9 contains the gene coding for an IFN-β wherein the five amino acids following the N-terminal Met [Ser Tyr Asn Leu Leu] are deleted.

Reference Example 1

Construction of a plasmid vector pKYP10 having a trp promoter

(a) Purification of tryptophan transducing phage DNA:

A λptrp phage, λc1857trpED10 (referred to as λtrp ED hereinafter) [G. F. Miazzari *et al.*: J. Bacteriol. *133*, 1457 (1978)] was lysogenized in the strain *Escherichia coli* JA 194 (F$^-$, λ$^-$, r$_k$$^-$, m$_k$$^-$, Δtrp E5, leu6) [J. Carbon *et al.*: Recombinant Molecules p. 355 (1977), Raven Press]. The resultant lysogenic strain JA 194 (λtrp ED) was cultured at 42°C for 30 minutes to induce λtrp ED phages and prepare a λphage lysate. The λphages were purified from the λphage lysate by the cesium chloride equilibrium density gradient centrifugation of Yamakawa *et al.*: "Chemicals of Nucleic Acids I" Tokyo Kagaku Dojin, p. 54—61 (1974). The DNA of the phages was purified by phenol treatment and chloroform treatment according to the method of Yamakawa *et al.*: "Chemicals of Nucleic Acids I" Tokyo Kagaku Dojin, p. 62—65 (1974).

(b) Cloning of the trp promoter into a plasmid:

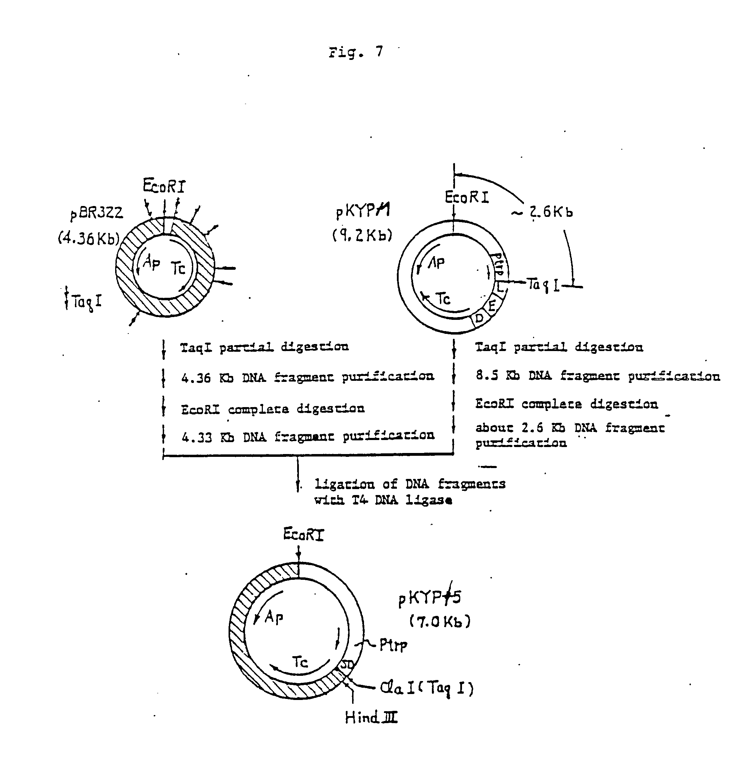

Cloning of the trp operon from λtrp ED phage DNA was carried out in the following manner. 8 µg of λtrp ED DNA were digested at 37°C for 2 hours with 16 units of EcoRI (product of Takara Shuzo Co., the same shall apply hereinafter) and 16 units of HindIII (product of Takara Shuzo Co., the same shall apply hereinafter) in 20 mM Tris-HCl (pH 7.5), 75 mM NaCl, 10 mM MgCl$_2$ and 5 mM dithiothreitol. Separately, 1 µg of plasmid pBR325 DNA was digested with 2 units of EcoRI and 2 units of HindIII by the same method as mentioned above (final volume: 30 µl). The reactions were stopped by heating at 65°C for 5 minutes. Then, 15 µl each of the digests were mixed and 500 µM (final concentration) ATP and 5 units of T4 DNA ligase (product of New England Biolabs) were added. The mixture was allowed to react at 4°C for 18 hours. *Escherichia coli* C600 SF strain, Cameron *et al.*: Proc. Natl. Acad. Sci. *72*, 3416 (1975) was transformed with the obtained plasmid mixture by the method of S. N. Cohen *et al.*: Proc. Natl. Acad. Sci. *69*, 2110 (1972) and transformants which were resistant to ampicillin (Ap$_R$), resistant to tetracycline (Tc$_R$) and sensitive to chloramphenicol (Cm$^S$) were selected. Plasmid DNAs were isolated from the transformants of *Escherichia coli*. One of the plasmid DNAs was named pKYP-1. The plasmid pKYP-1 was digested with TaqI and EcoRI and the digest was subjected to purification by agarose gel electrophoresis to obtain a 2.6 Kb (Kilobase; the same shall apply hereinafter) DNA fragment containing the tryptophan promoter and the SD sequence. The 2.6 Kb DNA fragment was cloned in a known vector, pBR322, by the method as illustrated in Fig. 7. That is, 8 µg of pBR322 were digested at 45°C for 60 minutes with 2 units of TaqI (product of Takara Shuzo Co.) in 100 µl of a reaction mixture comprising 10 mM Tris-HCl (pH 8.4), 6 mM MgCl$_2$, 100 mM NaCl and 6 mM 2-mercaptoethanol. After partial digestion with TaqI, the digest was subjected to low-gelling-temperature agarose gel electrophoresis [Lars Wieslander: Analytical Biochemistry *98*, 305 (1979] to obtain a purified 4.36 Kb DNA fragment. About 1.5 µg of the DNA fragment were completely digested at 37°C for 3 hours with 3 units of EcoRI. About 1.0 µg of an about 4.33 Kb DNA fragment was recovered by low-gelling-temperature agarose gel electrophoresis in a similar manner as above. Then, 12 µg of pKYP-1 DNA was partially digested with 3 units of TaqI, the digest was subjected to low-gelling-temperature agarose gel electrophoresis to obtain about 2 µg of a purified 8.5 Kb DNA fragment and the DNA was completely digested with EcoRI by the same procedure as above to obtain about 0.5 µg of a purified 2.6 Kb DNA fragment. Then, 0.4 µg of the 4.33 Kb DNA fragment of pBR322 and 0.25 µg of the 2.6 Kb DNA fragment of pKYP-1 thus obtained were added to 20 µl of a reaction solution consisting of 20 mM Tris-HCl (pH 7.6), 10 mM MgCl$_2$ and 10 mM MgCl$_2$ and 10 mM dithiothreitol. Then, 0.5 mM ATP and 4 units of T4 DNA ligase were added to the mixture and reaction was carried out at 4°C for 18 hours. *Escherichia coli* C600 SF8 strain was transformed with the recombinant plasmid DNA obtained as above. The plasmids of transformants being Ap$^R$ and Tc$^R$ were isolated. The plasmid DNA was digested with 6 restriction endonucleases, EcoRI, HindII, ClaI (product of Boehringer Mannheim GmbH), HpaI (product of Takara Shuzo Co.), HincII (product of Takara Shuzo Co.) and BamHI (product of Takara Shuzo Co.) to analyze the structure of the plasmid. The desired plasmid was named pKYP-5.

(c) Preparation of a portable promoter from the trp promoter:

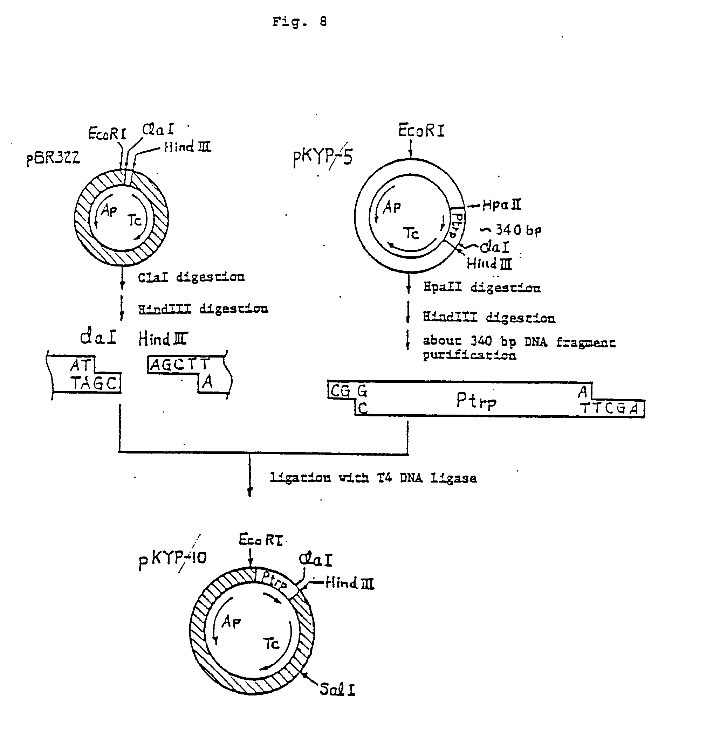

The plasmid vector pKYP-5 mentioned above is applicable as a DNA introducing vector since it has a ClaI site and a HindIII site on the DNA of 1 to 20 base pairs downstream from the SD sequence. However, another ClaI site is present in pKYP-5 DNA besides the ClaI site immediately after the SD sequence and the fragment with the trp promoter obtained by cutting pKYP-5 DNA wth EcoRI and HindIII is too large, i.e. 2.65 Kb. For convenience of use, pKYP-5 was improved by the process as illustrated in Fig. 8 to obtain a plasmid having a shorter DNA fragment containing the tryptophan promoter. That is, pKYP-5 DNA was digested with HpaII and HindIII and the digest was purified to obtain a DNA fragment of about 340 bp (base pairs). The fragment was inserted into pBR322 digested with ClaI and HindIII as illustrated in Fig. 6 to obtain pKYP-10. The structure of pKYP-10 was determined by agarose gel electrophoresis after the digestion with EcoRI, ClaI, HindIII and HpaI.

(d) Connection of two or more trp promoters:

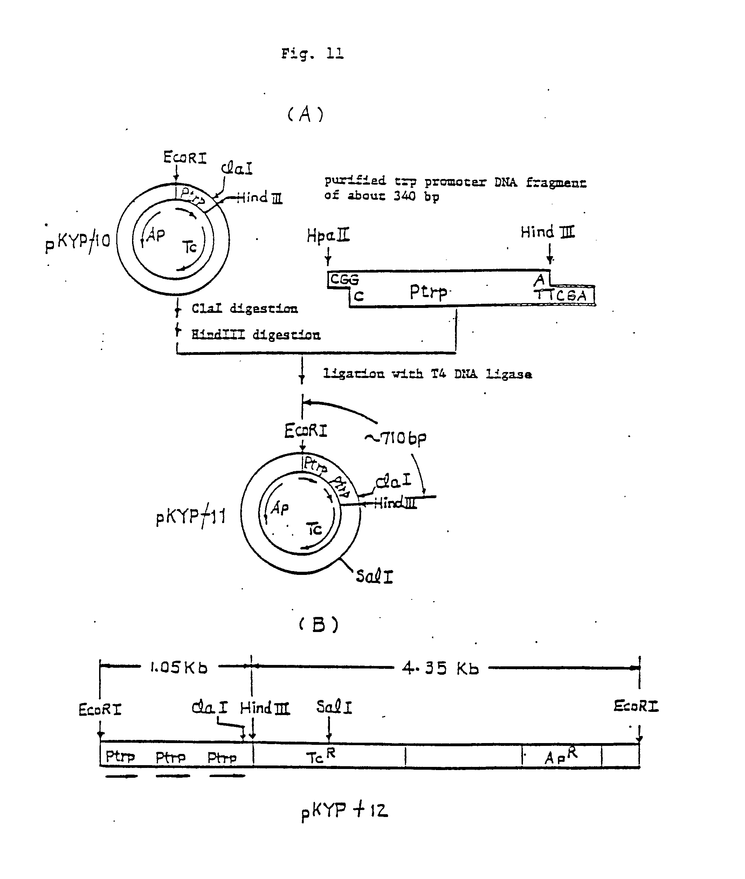

In order to construct a plasmid vector having a stronger promoter activity, two trp promoters were inserted into the vector. That is, the DNA fragment of about 340 bp containing the trp promoter mentioned in step (c) above was inserted into the pKYP-10 digested with ClaI and HindIII to obtain pKYP-11 as illustrated in Fig. 11 (A). The same procedure was repeated to construct pKYP-12 illustrated in Fig. 11 (B) wherein three trp promoters were connected at the same orientation. The structure of pKYP-12 was confirmed by digestion with EcoRI, ClaI, HindIII and HpaI.

Reference Example 2

Construction of pKYP100

pKYP100 *7* which is more convenient for the construction of the plasmid vector of the present invention was constructed as illustrated in Fig. 5. As the source of a DNA containing the trp promoter, plasmid pKYP10 *1* (Japanese Patent Application 213193/81, see Fig. 5) bearing a portable trp promoter was used.

50 µg of pKYP10 were digested with 50 units of HhaI (product of Takara Shuzo Co.) in 100 µl of a reaction solution containing 10 mM Tris-HCl (pH 7.5), 7 mM MgCl$_2$ and 6 mM 2-mercaptoethanol at 37°C for 2 hours. After digestion with HhaI, a DNA fragment of about 180 bp containing the trp promoter was purified by 5% polyacrylamide gel electrophoresis [A. M. Maxam *et al.*: Proc. Natl. Acad. Sci. *74*, 560 (1977), referred to as PAGE hereinafter]. In the purification step, two DNA fragments other than the desired DNA were obtained because of incomplete purification by PAGE. The three purified DNA fragments (total amount: about 4 µg) were allowed to react with 8 units of *Escherichia coli* DNA polymerase I·Klenow fragment (product of Bethesda Research Laboratories Inc.) in 30 µl of a reaction solution containing 50 mM Tris-HCl (pH 7.6), 7 mM MgCl$_2$, 10 mM 2-mercaptoethanol, 0.25 mM dATP, 0.25 mM dCPT, 0.25 mM dGTP and 0.25 mM dTTP at 15°C for 2 hours. By the reaction, the 3'-protruding ends formed by the HhaI digestion were converted into flush ends by the 3' → 5' exonuclease activity and 5' → 3' repairing synthesis activity of DNA polymerase I·Klenow fragment. Subsequently, the DNA polymerase I·Klenow fragment was inactivated by heating at 72°C for 30 minutes and the NaCl concentration was adjusted to 50 mM with 1 M NaCl. 8 units of HindIII (product of Takara Shuzo Co.) were added and the mixture was allowed to react at 37°C for 2 hours. After digestion with HindIII, the DNA fragment of about 100 bp containing trp promoter was isolated and purified by PAGE.

Separately, 5 µg of plasmid pBR322 were digested with 8 units of EcoRI (product of Takara Shuzo Co.) in 20 µl of a reaction solution containing 10 mM Tris-HCl (pH 7.5), 50 mM NaCl, 7 mM MgCl$_2$ and 6 mM 2-mercaptoethanol at 37°C for 2 hours. After phenol and chloroform extraction and ethanol precipitation, the precipitated DNA fragment was dissolved in 20 µl of a mixture of 50 mM Tris-HCl (pH 7.6), 7 mM MgCl$_2$, 6 mM 2-mercaptoethanol, 0.25 mM dATP, 0.25 mM dCTP, 0.25 mM dGTP and 0.25 mM dTTP. Then, 8 units of *Escherichia coli* DNA polymerase I·Klenow fragment (produced of Bethesda Research Laboratories Inc.) were added and the mixture was allowed to react at 15°C for 2 hours. The 5'-protruding ends formed by the EcoRI digestion were converted into flush ends by the repairing synthesis activity of the DNA polymerase I·Klenow fragment.

The DNA polymerase I·Klenow fragment was inactivated by heating at 72°C for 30 minutes and the NaCl concentration was adjusted to 50 mM with 1 M NaCl. 8 units of HindIII (product of Takara Shuzo Co.) were added and the mixture was allowed to react at 37°C for 2 hours. After digestion with HindIII, the larger plasmid DNA fragment of about 4.33 Kb was purified by low-gelling-temperature agarose gel electrophoresis.

About 50 ng of the DNA fragment *3* of about 100 bp containing trp promoter and obtained above, about 0.2 µg of the DNA fragment *6* of about 4.33 Kb derived from pBR322 and obtained above, and 50 g of 5'-phosphorylated XhoI linker (pCCTCGAGG, product of Collaborative Research) were ligated with 1 unit of T4

# EP 0 121 569 B1

DNA ligase (product of Takara Shuzo Co.) in 20 µl of a reaction solution containing 20 mM tris-HCl (pH 7.6), 10 mM dithiothreitol and 0.5 mM ATP at 4°C for 40 hours. *Escherichia coli* HB101 was transformed with the thus obtained recombinant plasmid DNA and plasmid DNAs were isolated and purified from the $Ap^R Tc^R$ transformants. These plasmid DNAs were digested with 8 restriction enzymes EcoRI, XhoI, HindIII and HaeIII (products of Takara Shuzo Co.), ClaI (product of Boehringer Mannheim GmbH), TaqI (product of Bethesda Research Laboratories) and RsaI (product of New England Biolabs) to select a plasmid wherein the DNA fragment 3 of about 100 bp containing the trp promoter and the XhoI linker 4 were cloned, which was named pKYP100 7.

Reference Example 3

Construction of pLE-3

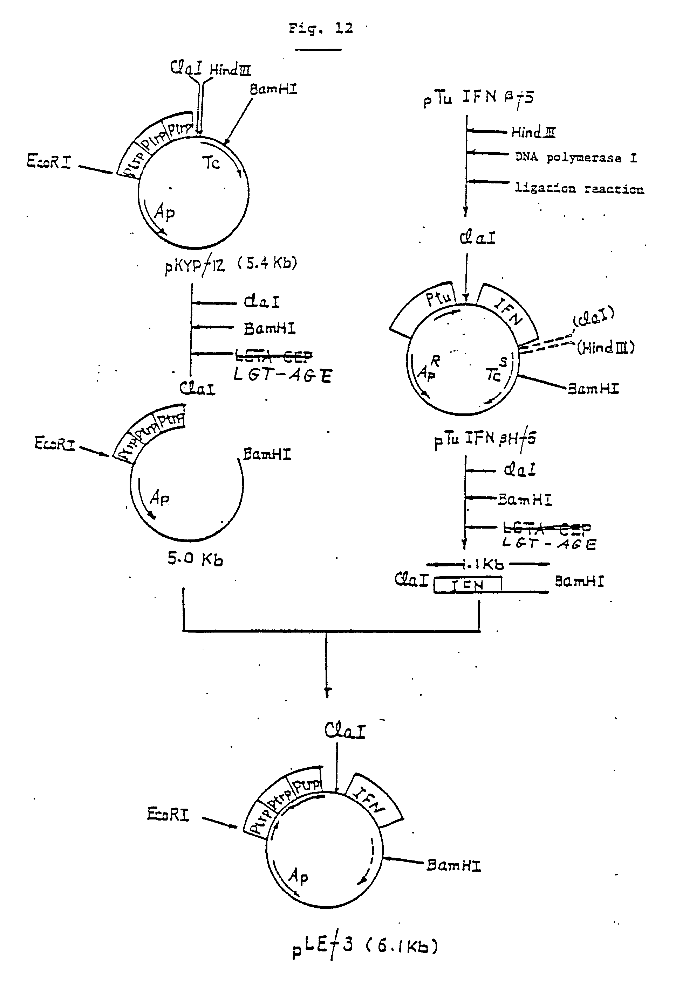

Plasmid pTuIFNβ-5, isolated from ATCC 31879 by conventional techniques, was digested with HindIII. The digest was treated with DNA polymerase I (product of New England Biolabs) and subjected to ligation to obtain pTuIFNβH-5 illustrated in Fig. 12. The IFN-β gene recovered from pTuIFNβH-5 was then cloned in the plasmid pKYP-12 having a trp promoter. That is, 2 µg of pKYP-12 DNA were digested with 4 units of ClaI in 30 µl of a buffer solution consisting of 10 mM Tris-HCl (pH 7.5), 6 mM $MgCl_2$ and 5 mM dithiothreitol (referred to as Cla buffer hereinafter) at 37°C for 2 hours. NaCl was added to a final concentration of 100 mM and the reaction was continued at 37°C for 2 hours. The mixture was heated at 65°C for 5 minutes to inactivate the enzyme and was then subjected to low-gelling-temperature agarose gel electrophoresis to obtain 1.2 µg of a purified DNA fragment of about 5 Kb containing the trp promoter. Separately, 15 µg of pTuIFNβH-5 DNA was digested with ClaI and BamHI, and the digested DNA was purified by low-gelling-temperature agarose gel electrophoresis as described above to obtain about 1 µg of a DNA fragment (1.1 Kb) containing the IFN-β gene. The two DNA fragments obtained as above and illustrated as 5 Kb and 1.1 Kb in Fig. 12 were dissolved in a mixture of 20 mM Tris-HCl (pH 7.5), 6 mM $MgCl_2$, 5 mM dithiothreitol and 500 µM ATP. 4 units of T4 DNA ligase were added and the mixture was allowed to react at 4°C for 18 hours. *Escherichia coli* HB101 was transformed with the thus obtained mixture of the recombinants by conventional techniques to obtain $Ap^R$ colonies. Plasmids were recovered from the culture of the colonies. One of the plasmids is pLE3 as illustrated in Fig. 12. The structure of pLE-3 was confirmed by agarose gel electrophoresis after the digestion of DNA with ClaI, EcoRI, HindIII and BamHI. It was confirmed by the method of Maxam & Gilbert [A. M. Maxam, *et al.*, Proc. Natl. Acad. Sci. *74*, 560 (1977)] that the nucleotide sequence from the SD sequence, AAGG, to the initiation codon, ATG, in the plasmid pLE-3 was "*AAGG*GTATCG*ATG*".

*Escherichia coli* containing the plasmid pLE-3 has been deposited with the American Type Culture Collection (ATCC) as *Escherichia coli* ILE-3, ATCC 39010.

## Claims

1. An expression vector having a promoter, a ribosome binding site, an initiation codon for translation and a recognition sequence of a restriction enzyme to be left as 3'-protruding ends after cleavage with the restriction enzyme, characterized in that the recognition sequence is situated immediately downstream of the initiation codon.

2. The expression vector according to Claim 1, wherein the initiation codon for translation is ATG or GTG.

3. The expression vector according to Claim 1, wherein the restriction enzyme is SphI, KpnI or SacI.

4. The expression vector according to Claim 1, wherein the promoter is derived from a prokaryote.

5. The expression vector according to Claim 4, wherein the promoter is an *Escherichia coli* promoter selected from trp, lac and λphage or a *Bacillus subtilis* promoter.

6. A process for expressing a foreign gene which comprises cleaving with a restriction enzyme producing 3'-protruding ends an expression vector according to any one of claims 1 to 5 changing the 3'-protruding ends to a flush end with DNA polymerase I (Klenow fragment) or T4 DNA polymerase, incorporating the foreign gene into the flush ends to form a recombinant expression plasmid, transforming a host microorganism with the recombinant expression plasmid and culturing the thus obtained transformant.

7. A process according to claim 6 wherein instead of producing flush ends a homopolymer block is attached to the 3'-protruding ends using a terminal deoxynucleotidyl transferase, and wherein the foreign gene to be inserted contains at its ends a homopolymer block complementary to the above homopolymer block.

8. A microorganism containing the expression vector according to claim 1.

9. *Escherichia coli* ITrS-3 (Ferm B-328).

10. The process according to claim 6, wherein the foreign gene is a gene coding for interferon or a derivative thereof.

11. The process according to claim 6, wherein the host microorganism belongs to the species *Escherichia coli*.

12. *Escherichia coli* IFJ-123 (FERM BP-346).

13. *Escherichia coli* IFM-9 (FERM BP-347).

9

# EP 0 121 569 B1

**Patentansprüche**

1. Expressionsvektor mit einem Promotor, einer Ribosomen-Bindungsstelle, einem Startcodon für die Translation und einer Erkennungssequenz für ein Restriktionsenzym, die als 3'-vorstehende Enden nach Spaltung mit dem Restriktionsenzym verbleiben soll, dadurch gekennzeichnet, daß sich die Erkennungssequenz unmittelbar nach dem Startcodon befindet.

2. Expressionsvektor nach Anspruch 1, wobei das Startcodon für die Translation ATG oder GTG ist.

3. Expressionsvektor nach Anspruch 1, wobei das Restriktionsenzym Sphl, Kpnl oder Sacl ist.

4. Expressionsvektor nach Anspruch 1, wobei der Promotor von einem Prokaryonten abgeleitet ist.

5. Expressionsvektor nach Anspruch 4, wobei der Promotor ein *Escherichia coli*-Promotor, ausgewählt von trp, lac und lambda-Phage, oder ein *Bacillus subtilis*-Promotor ist.

6. Verfahren zur Expression eines fremden Gens, welches die Spaltung eines Expressionsvektors nach einem der Ansprüche 1 bis 5 mit einem 3'-vorstehende Enden erzeugenden Restriktionsenzym, Umwandlung der 3'-vorstehenden Enden in glatte Enden mit DNA-Polymerase I (Klenow-Fragment oder T4-DNA-Polymerase, Einbau des fremden Gens in die glatten Enden zur Erzeugung eines rekombinanten Expresssionsplasmids, Transformierung eines Wirtsmikroorganismus mit dem rekombinanten Expressionsplasmid und Züchtung der so erhaltenen Transformante umfaßt.

7. Verfahren nach Anspruch 6, wobei anstelle der Erzeugung von glatten Enden ein Homopolymerblock an die 3'-voststehenden Enden unter Verwendung einer terminalen Deoxynucleotidyl-Transferase angefügt wird, und wobei das fremde, einzufügende Gen an seinen Enden einen zu dem vorstehend genannten Homopolymerblock komplementären Homopolymerblock aufweist.

8. Mikroorganismus, enthaltend einen Expressionsvektor nach Anspruch 1.

9. *Escherichia coli* ITrS-3 (FERM BP-328).

10. Verfahren nach Anspruch 6, wobei das fremde Gen ein für Interferon oder ein Derivat davon codierendes Gen ist.

11. Verfahren nach Anspruch 6, wobei der Wirtsmikroorganismus zur Gattung *Escherichia coli* gehört.

12. *Escherichia coli* IFJ-123 (FERM BP-346).

13. *Escherichia coli* IFM-9 (FERM BP-347).

**Revendications**

1. Vecteur d'expression comportant un promoteur, un site de liaison au ribosome, un codon d'initiation de traduction et une séquence de reconnaissance d'une enzyme de restriction, devant être laissée comme extrémités cohésives 3'-terminales après coupure par l'enzyme de restriction, caractérisé en ce que la séquence de reconnaissance est située immédiatement en aval du codon d'initiation.

2. Vecteur d'expression conforme à la revendication 1, dans lequel le codon d'initiation de traduction est ATG ou GTG.

3. Vecteur d'expression conforme à la revendication 1, pour lequel l'enzyme de restriction est Sphl, Kpnl ou Sacl.

4. Vecteur d'expression conforme à la revendication 1, dans lequel le promoteur provient d'un procaryote.

5. Vecteur d'expression conforme à la revendication 4, dans lequel le promoteur est un promoteur d'*Escherichia coli* choisi parmi trp, lac et phage λ, ou un promoteur de *Bacillus subtilis*.

6. Procédé d'expression d'un gène étranger qui comporte la coupure, par une enzyme de restriction produisant des extrémités 3'-terminales cohésives, d'un vecteur d'expression conforme à l'une quelconque des revendications 1 à 5, la transformation des extrémités 3'-terminales cohésives an extrémités franches par l'ADN polymérase I (fragment de Klenow) ou l'ADN polymérase de T4, l'incorporation du gène étranger au niveau des extrémités franches pour former un plasmide recombinant d'expression, la transformation d'un microorganisme hôte avec le plasmide recombinant d'expression et le culture du transformant ainsi obtenu.

7. Procédé conforme à la revendication 6, dans lequel, au lieu de la production d'extrémités franches, une séquence homopolymère est liée aux extrémités 3'-terminales cohésives à l'aide d'une désoxy-nucléotidyltransférase terminale, et dans lequel le gène étranger à insérer contient, au niveau de ses extrémités, une séquence homopolymère complémentaire de la séquence homopolymère mentionnée ci-dessus.

8. Microorganisme contenant le vecteur d'expression conforme à la revendication 1.

9. *Escherichia coli* ITrS-3 (FERM BP-328).

10. Procédé conforme à la revendication 6, dans lequel le gène étranger est un gène codant pour l'interféron ou l'un de ses dérivés.

11. Procédé conforme à la revendication 6, dans lequel le microorganisme hôte appartient à l'espèce *Escherichia coli*.

12. *Escherichia coli* IFJ-123 (FERM BP-346).

13. *Escherichia coli* IFM-9 (FERM BP-347).

10

Fig. 1

```
                                                              SphI
                                                               |
                                                               |
─────────────────────────────────────────────────────────────┬───────
          |         |  Ribosome  |           A T G C A T G C
 Promoter |         |  binding   |           T A C G T A C G
          |         |  site      |                    ↑
═════════════════════════════════════════════════════════════════════
```

Fig. 2

```
                    ↓
                    |
          G C A T G'C
SphI :    C G T A C G
            ↑
            |
                      |
                      ↓
          G G T A C'C              G A G C T'C
KpnI :    C C A T G G      SacI :  C T C G A G
          ↑                        ↑
          |                        |
```

Fig. 3

Fig. 4

SphI

Gene coding for polypeptide

| Promoter | | SD | ATGCATG C<br>TAC GTAC G |

G G G G G ▨▨▨▨▨ G G G G G

SphI

| Promoter | | SD | ATGCATG<br>TAC | C<br>GTACG |

terminal transferase

| Promoter | | SD | ATGCATG C C C C C<br>TAC | C C C C C GTACG C |

| Promoter | | SD | ATGCATGCCC ⋯ CCC ▨▨▨▨▨ GGG ⋯ GGGCATGC<br>TACGTACGGG ⋯ GGG ▨▨▨▨▨ CCC ⋯ CCCGTACG |

EP 0 121 569 B1

Fig. 5

Fig. 6

Fig. 7

pBR3ZZ
(4.36 Kb)

EcoRI

Ap Tc

TaqI

TaqI partial digestion

4.36 Kb DNA fragment purification

EcoRI complete digestion

4.33 Kb DNA fragment purification

pKYP1
(9.2 Kb)

EcoRI ~2.6Kb

Ap

Tc

TaqI

TaqI partial digestion

8.5 Kb DNA fragment purification

EcoRI complete digestion

about 2.6 Kb DNA fragment purification

Ligation of DNA fragments with T4 DNA ligase

EcoRI

Ap

Tc

Ptrp

SD

ClaI(TaqI)

HindⅢ

pKYP5
(7.0 Kb)

Fig. 8

Fig. 9

Fig. 10

Fig. 11

(A)

(B)

pKYP↑12

Fig. 12